# EUROPÄISCHE PATENTANMELDUNG

(11) **EP 0 582 929 A1**
(43) Veröffentlichungstag der Anmeldung: **16.02.1994**
(21) Anmeldenummer: 93112346.7
(22) Anmeldetag: 02.08.1993
(51) Int. Cl.: C07C 213/02, C07C 219/34

(54) **Verfahren zur Herstellung von Aminophenylacetaten**

(30) Priorität: 08.08.1992 DE 4226280
(71) Anmelder: HOECHST AKTIENGESELLSCHAFT, D-65926 Frankfurt am Main (DE)
(72) Erfinder: Beller, Matthias, Dr., D-65527 Niedernhausen (DE); Strutz, Heinz, Dr., D-61250 Usingen (DE)

(57) **Zusammenfassung**

Die vorliegende Erfindung betrifft ein Verfahren zur Herstellung eines Aminophenylacetats oder Aminophenyldiacetats, indem man ein Nitrophenylacetat oder Nitrophenyldiacetat in Gegenwart eines Hydrierkatalysators mit Wasserstoff bei 0 bis 60 °C und wenigstens 4 MPa H₂-Druck in Anwesenheit eines Lösungsmittels oder Lösungsmittelgemisches umsetzt.

## Beschreibung

Die vorliegende Erfindung betrifft ein gegenüber dem Stand der Technik verbessertes Verfahren zur Herstellung von Aminophenylacetaten durch Umsetzung der entsprechenden Nitrophenylacetate mit Wasserstoff in Anwesenheit eines Hydrierkatalysators.

Aufgrund ihrer funtionellen Gruppen sind Aminophenylacetate generell und insbesondere 4-Aminophenylacetat geeignete Ausgangsstoffe für eine Vielzahl verschiedener chemischer Verbindungen. Sie dienen beispielsweise als vielseitig verwendbare Vorstufen für die Heck-Reaktion von in situ dargestellten Diazoniumsalzen, wie aus K. Kikukawa et al., J. Org. Chem. 1981, 46, 4885 zu entnehmen ist. Ferner können Aminophenylacetate als Vorstufen zur Herstellung einer großen Zahl von Feinchemikalien, Pflanzenschutzmitteln und Pharmazeutika eingesetzt werden.

Die in der Literatur bisher beschriebenen Verfahren zur Herstellung von Aminophenylacetatderivaten sind zumindest zum Teil recht aufwendig und daher technisch nur sehr schwer zu realisieren und/oder liefern die gewünschten Wertprodukte lediglich in geringen Ausbeuten.
So läßt sich nach Hazlet und Dornfeld, J. Am. Chem. Soc. 1944, 66, 1781 4-Aminophenylacetat durch Reduktion von 4-Nitrophenylacetat mit Eisen und einer Mineralsäure herstellen. Die Ausbeute beträgt allerdings nur 9 - 13 %.

Einen anderen Weg beschreibt L. Galatis, Chem. Ber. 1926, 59, 848. Man setzt p-Aminophenol mit Benzaldehyd zu der entsprechenden Benzyliden-Verbindung um, die anschließend einer O-Acetylierung unterzogen und in kalter Mineralsäure hydrolysiert wird. Die Ausbeute ausschließlich der letzten Stufe beträgt nur etwa 35 %, hingegen ist die Ausbeute für die jeweils vorausgegangenen Stufen nicht angegeben.

Eine neuere Synthese geht von 4-Aminophenol aus, das selektiv mit Acetanhydrid in Anwesenheit von Cobalt(II)chlorid zu der entsprechenden O-Acetylverbindung umgesetzt wird (S. Ahmad, J. Iqual, J. Chem. Soc. Chem. Comm. 1987, 114). Die Ausbeute beträgt 83 %. Dieses Verfahren weist jedoch Nachteile auf. Einerseits ist es erforderlich, unter striktem Ausschluß von Wasser zu arbeiten, und andererseits existiert der Zwang, die Umsetzung unter einer inerten Schutz-Gas-Atmosphäre durchzuführen.

Eine weitere von 4-Nitrophenylacetat ausgehende Synthese ist aus Satoh et al., Chem. Pharm. Bull. 1981, 29, 1443 bekannt. Hierbei wird 4-Nitrophenylacetat mit Zinn(II)chlorid im Überschuß und Natriumborhydrid in Anwesenheit eines aliphatischen Alkohols als Solvens umgesetzt. Dieses Verfahren ist allerdings aufgrund seines hohen Bedarfs an Zinn(II)chlorid und wegen des Gebrauches von teurem Natriumborhydrid zwar für ein Laborverfahren, nicht jedoch für ein technisches Verfahren geeignet.

Wie aus R. Feldstein et. al., J. Org. Chem. 1961, 26, 1656 hervorgeht, entsteht bei der Umsetzung von 4-Nitrophenylacetat mit Wasserstoff in Anwesenheit von Platinoxid als Katalysator nicht p-Aminophenylacetat, sondern es bildet sich vermutlich unter Wanderung des Acetylrestes das entsprechende p-Acetamidophenol in einer Ausbeute von nicht weniger als 77 %.

In diesem Zusammenhang ist auch die von J. E. Wynn et al., J. Pharm. Sci. 1982, 7, 772 beschriebene Umsetzung von p-Nitrophenylacylestern mit Wasserstoff in Anwesenheit von PtO₂ als Katalysator in Ethanol zu sehen. Diese Umsetzung führt bei Einsatz von p-Nitrophenylacetat unter Verwendung von PtO₂ als Katalysator und absolutem Ethanol als Lösungsmittel lediglich zu einer Ausbeute von 43 % p-Aminophenylacetat.
Die Hydrierung substituierter Nitrophenylacetate ist nicht beschrieben.

Die generellen Schwierigkeiten, Aminophenylacetate und deren Derivate zu synthetisieren, sind auf die möglicherweise durch die Anwesenheit einer Aminogruppe verursachte Instabilität der Phenylestergruppe zurückzuführen. Offensichtlich wandeln sich die Aminophenylacetate in Lösung allmählich unter Wanderung der Acetylgruppe in die thermodynamisch stabileren Acetamidophenole um. Aus der EP-0 435 263 A1 geht hervor, daß Spuren von Säuren oder Basen diesen Prozess katalysieren.

Wie die vorangegangenen Ausführungen belegen, besteht ein Bedarf bezüglich eines Verfahrens, das die vorstehend geschilderten Schwierigkeiten vermeidet, sich zudem auch im technischem Maßstab anwenden läßt, sich einfach realisieren läßt und sich technisch leicht zugänglicher Hilfsstoffe bedient. Das Verfahren soll weiterhin das gewünschte Wertprodukt in hoher Ausbeute zugänglich machen und dabei die Entstehung von Nebenprodukten möglichst weitgehend vermeiden.

Gelöst wird diese Aufgabe durch ein Verfahren zur Herstellung eines Aminophenylacetats oder Aminophenyldiacetats. Es ist dadurch gekennzeichnet, das man ein Nitrophenylacetat oder ein Nitrophenyldiacetat in Gegenwart eines Hydrierkatalysators mit Wasserstoff bei 0 - 60 °C und wenigstens 4 MPa H₂-Druck in Anwesenheit eines Lösungsmittels oder Lösungsmittelgemisches umsetzt.

Im Hinblick auf die von R. Feldstein et al., J. Org. Chem. 1961, 26, 1656 und J. E. Wynn et al., J. Pharm. Sci. 1982, 7, 772 mittels katalytischer Hydrierung erzielten Ergebnisse war es überraschend, daß das erfindungsgemäße Verfahren Aminophenylacetate durch katalytische Hydrierung entsprechender Nitrophenylacetate in hohen Ausbeuten liefert.

Als Nitrophenylacetate eignen sich sowohl Nitrophenylacetate oder Nitrophenyldiacetate, die am aromatischen Kern nicht substitutiert sind, als auch Nitrophenylacetate oder Nitrophenyldiacetate, die am aromatischen Kern einfach oder mehrfach substituiert sind.

Üblicherweise setzt man als Nitrophenylacetat ein gegebenenfalls am aromatischen Kern einfach oder mehrfach durch eine Alkylgruppe mit 1 bis 12, insbesondere 1 bis 4 Kohlenstoffatomen, durch ein Halogenatom, durch eine Hydroxygruppe, durch eine Alkylethergruppe mit 1 bis 4 Kohlenstoffatomen im Alkylrest, durch eine Arylethergruppe, durch eine Nitrilgruppe, durch eine RS-, durch eine R₃Si- oder R-CO-Gruppe, worin R für einen Alkylrest mit 1 bis 4 Kohlenstoffatomen oder eine Arylethergruppe steht, durch eine Sulfonsäuregruppe, durch eine Phosphonsäuregruppe, durch eine Sulfoxidgruppe, durch eine Carbonsäuregruppe und/oder Carbonsäuresalzgruppe substituiertes Nitrophenylacetat ein.

Es ist zweckmäßig, als Nitrophenylacetat ein gegebenenfalls am aromatischen Kern einfach oder mehrfach durch eine Alkylgruppe mit 1 bis 12, insbesondere 1 bis 4 Kohlenstoffatomen, durch ein Halogebatom, durch eine Hydroxygruppe, durch eine Alkylethergruppe mit 1 bis 4 Kohlenstoffatomen im Alkylrest, durch eine Arylethergruppe, durch eine Nitrilgruppe, durch eine R-CO-Gruppe, worin R für einen Alkylrest mit 1 bis 4 Kohlenstoffatomen steht, oder durch eine Carbonsäuregruppe, insbesondere durch eine Alkylgruppe mit 1 bis 12, bevorzugt 1 bis 4 Kohlenstoffatomen, durch ein Halogenatom, durch eine Alkylethergruppe mit 1 bis 4 Kohlenstoffatomen im Alkylrest, durch eine Nitrilgruppe oder eine R-CO-Gruppe, worin R für einen Alkylrest mit 1 bis 4 Kohlenstoffatomen steht, bevorzugt durch eine Alkylgruppe mit 1 bis 12, insbesondere mit 1 bis 4 Kohlenstoffatomen, oder durch eine Alkylethergruppe mit 1 bis 4 Kohlenstoffatomen im Alkylrest substituiertes Nitrophenylacetat einzusetzen.

Geeignet als Einsatzstoff sind ein gegebenenfalls am aromatischen Kern einfach oder mehrfach substituiertes o-Nitrophenylacetat, m-Nitrophenylacetat oder p-Nitrophenylacetat, insbesondere o-Nitrophenylacetat oder p-Nitrophenylacetat bevorzugt p-Nitrophenylacetat.

Gut geeignete Nitrophenylacetate sind o-Nitrophenylacetat, m-Nitrophenylacetat, p-Nitrophenylacetat oder 2-Methyl-4-Nitrophenylacetat, insbesondere p-Nitrophenylacetat oder 2-Methyl-4-Nitrophenylacetat.

Zur Durchführung der Reaktion können übliche Hydrierkatalysatoren verwendet werden. Die Hydrierkatalysatoren enthalten in der Regel Kupfer, Mangan, Cobalt, Nickel, Rhodium, Platin und/oder Palladium, sowie gegebenenfalls geeignete Aktivatoren und Promotoren.

Recht gut eignen sich Cobalt, Nickel, Platin und/oder Palladium enthaltende Hydrierkatalysatoren, die gegebenenfalls geeignete Aktivatoren und Promotoren enthalten.
Besonders geeignet sind Nickel und/oder Palladium enthaltende Katalysatoren, die gegebenenfalls geeignete Aktivatoren und Promotoren enthalten.

Es ist möglich, das erfindungsgemäße Verfahren sowohl mittels eines trägerfreien Katalysators als auch unter Verwendung eines Trägerkatalysators durchzuführen.

Verwendet man einen trägerfreien Katalysator, so empfehlen sich Raney-Cobalt oder Raney-Nickel, insbesondere Raney-Nickel. Die Trägerkatalysatoren enthalten die üblicherweise verwendeten Trägermaterialien. Geeignete Trägermaterialien sind Al₂O₃, Tonerde, Bimsstein, SiO₂, Silicagel, Kieselsäure, Kieselgur und/oder Aktivkohle, insbesondere Al₂O₃, Tonerde, Kieselgur und/oder Aktivkohle, bevorzugt Al₂O₃, Kieselgur und/oder Aktivkohle.

Besonders einfach gestaltet sich das erfindungsgemäße Verfahren unter Verwendung von Trägerkatalysatoren. Gut geeignet sind edelmetallhaltige Trägerkatalysatoren sowie Cobalt und Nickel enthaltende Trägerkatalysatoren. Als edelmetallhaltige Trägerkatalysatoren haben sich Platin auf Aktivkohle und/oder Palladium auf Aktivkohle, insbesondere Palladium auf Aktivkohle bewährt. Es lassen sich jedoch auch Platin auf Al₂O₃ und/oder Palladium auf Al₂O₃, insbesondere Palladium auf Al₂O₃ verwenden. Gut geeignet sind auch Cobalt und/oder Nickel, insbesondere Nickel enthaltende Trägerkatalysatoren, die beispielsweise Tonerde, SiO₂, Silicagel, Kieselsäure und/oder Kieselgur, insbesondere Silicagel, Kieselsäure und/oder Kieselgur, bevorzugt Kieselgur als Trägermaterial enthalten.

Das Verfahren läßt sich sowohl diskontinuierlich als auch kontinuierlich durchführen. Bei diskontinuierlicher Arbeitsweise empfiehlt es sich, den Katalysator in zerkleinertem Zustand zu suspendieren und die Reaktion mit Hilfe des suspendierten Katalysators durchzuführen. Bei kontinuierlicher Arbeitsweise empfiehlt es sich, den Katalysator beispielsweise in stückiger Form als Festbett angeordnet zu benutzen. Üblicherweise befindet sich der als Festbett angeordnete Katalysator in einem senkrecht stehenden Rohr. Je nach Art der Zuführung des zu hydrierenden Einsatzstoffes unterscheidet man zwischen einer Rieselfahrweise und einer Sumpffahrweise. Bei der Rieselfahrweise wird das zu hydrierende Einsatzmaterial über Kopf dem als Festbett angeordneten Katalysator zugeleitet, während der Wasserstoff entweder im Gleichstrom von oben nach unten oder im Gegenstrom von unten nach oben geführt wird. Bei der Sumpffahrweise wird der zu hydrierende Einsatzstoff von unten nach oben durch das Katalysator enthaltende Festbett geführt. Der Wasserstoff wird hierbei üblicherweise im Gleichstrom geleitet.

Das erfindungsgemäße Verfahren wird bei einer Temperatur von 0 bis 60 °C durchgeführt.
Die anzuwendende Reaktionstemperatur richtet sich im gewissen Umfange auch nach der Reaktivität des Nitrophenylacetats oder Nitrophenyldiacetats. Relativ reaktionsfreudige Nitrophenylacetate oder Nitrophenyldiacetate ermöglichen eine Arbeitsweise bei relativ niedrigen Temperaturen, hingegen bedingen vergleichsweise reaktionsträge Nitrophenylacetate oder Nitrophenyldiacetate entsprechend höhere Temperaturen.
Neben der Reaktivität des Einsatzmaterials beeinflußt auch die Aktivität des Hydrierkatalysators die anzuwendende Reaktionstemperatur in einem gewissen Ausmaße. Aktive Hydrierkatalysatoren erlauben es, die Reaktion bei vergleichsweise niedrigeren Temperaturen durchzuführen, während weniger aktive Hydrierkatalysatoren eine Durchführung der Reaktion bei höheren Temperaturen erfordern.

In vielen Fällen hat es sich bewährt, die Umsetzung bei 10 bis 50, insbesondere 15 bis 45, bevorzugt 20 bis 40 °C durchzuführen.
Um das erfindungsgemäße Verfahren mit Erfolg auszuüben, ist es erforderlich, einen bestimmten H₂-Druck einzuhalten. Der H₂-Druck soll wenigstens 4 MPa betragen.

Die Wahl des H₂-Druckes hängt im gewissen Umfange von der gewählten Reaktionstemperatur, von der Reaktivität des Einsatzmaterials und von der Aktivität des Hydrierkatalysators ab. So erlaubt eine vergleichsweise hohe Reaktionstemperatur und/oder ein vergleichsweise reaktives Nitrophenylacetat bzw. Nitrophenyldiacetat und/oder ein vergleichsweise aktiver Hydrierkatalysator eine Arbeitsweise bei relativ niedrigen Drücken. Hingegen empfiehlt es sich bei Anwendung einer relativ niedrigen Temperatur und/oder Verwendung eines relativ reaktionsträgen Nitrophenylacetats bzw. Nitrophenyldiacetats und/oder Gebrauch eines Hydrierkatalysators mit geringerer Aktivität eine Arbeitsweise bei relativ höheren Drücken.

In den meisten Fällen reicht es aus, die Umsetzung bei 4 bis 100, insbesondere 5 bis 50, bevorzugt 5 bis 30 MPa H₂-Druck durchzuführen.

Man löst das Nitrophenylacetat oder Nitrophenyldiacetat in einem Lösungsmittel oder Lösungsmittelgemisch und verwendet diese Lösung für die katalytische Umsetzung mit Wasserstoff.
Als Lösungsmittel setzt man einen geradkettigen und/oder verzweigten, einwertigen und/oder mehrwertigen aliphatischen Alkohol, insbesondere einen verzweigten einwertigen Alkohol, ein aliphatisches Keton, einen nichtcyclischen und/oder cyclischen aliphatischen Ether, einen Alkylester einer aliphatischen Carbonsäure, ein Alkylbenzol und/oder ein Dialkylamid einer aliphatischen Carbonsäure ein.

Es hat sich bewährt, als Lösungsmittel einen aliphatischen Alkohol mit 3 bis 8 Kohlenstoffatomen, insbesondere Isopropanol und/oder sek.-Butanol und/oder tert.-Butanol, ein aliphatisches Keton mit 3 bis 8 Kohlenstoffatomen, insbesondere Aceton und/oder Methylethylketon, einen nichtcyclischen aliphatischen Ether mit 2 bis 4 Kohlenstoffatomen je Alkylgruppe, insbesondere Diethylether und/oder Di-n-butylether, einen cyclischen aliphatischen Ether mit 4 bis 5 Kohlenstoffatomen im Ring, insbesondere Tetrahydrofuran und/oder Dioxan, einen Alkylester einer aliphatischen Carbonsäure mit 1 bis 4 Kohlenstoffatomen, insbesondere einen Methylester oder Ethylester der Ameisensäure und/oder Essigsäure, ein Alkylbenzol wie Toluol, Xylol und/oder Isopropylbenzol, insbesondere Toluol, und/oder ein Dialkylamid einer aliphatischen Carbonsäure mit 1 bis 4 Kohlenstoffatomen, insbesondere Dimethylformamid, Dimethylacetamid und/oder N-Methylpyrrolidin zu gebrauchen.
Methanol und/oder Ethanol sind als Lösungsmittel nicht geeignet, wenn sie als einziges Lösungsmittel, d.h. ohne weiteren Zusatz eines Solvens verwendet werden. Möglicherweise begünstigen Methanol und/oder Ethanol die unter Wanderung des Acetylrestes ablaufende Bildung von Acetamidophenolen.

Als Lösungsmittelgemisch verwendet man ein aus einem protisch polaren Lösungsmittel und einem aprotischen Lösungsmittel bestehendes Lösungsmittelgemisch.
Üblicherweise setzt man als Lösungsmittelgemisch ein protisch polares Lösungsmittel, insbesondere einen geradkettigen oder verzweigten aliphatischen Alkohol mit 1 bis 4 Kohlenstoffatomen und ein aprotisches Lösungsmittel, insbesondere Tetrahydrofuran und/oder Dioxan ein. Mit besonders gutem Erfolg läßt sich ein aus Isopropanol und Tetrahydrofuran bestehendes Lösungsmittelgemisch verwenden.

Als Lösungsmittelgemisch setzt man üblicherweise 5 bis 95, insbesondere 10 bis 90, bevorzugt 20 bis 80 Gewichtsteile protisch polares Lösungsmittel und 95 bis 5, insbesondere 90 bis 10, bevorzugt 80 bis 20 Gewichtsteile aprotisch polares Lösungsmittel ein.
In vielen Fällen hat sich ein aus 30 bis 70 Gewichtsteilen protisch polarem Lösungsmittel und aus 70 bis 30 Gewichtsteilen aprotisch polarem Lösungsmittel bestehendes Lösungsmittelgemisch bewährt.

Die nachfolgende Beispiele belegen die Erfindung, ohne sie zu beschränken.

### Experimenteller Teil

### Beispiel 1

In einem Autoklaven (Volumen 250 ml) werden 8,0 g p-Nitrophenylacetat (4-Nitrophenylacetat) in einem Gemisch von 60 ml Isopropanol und 30 ml Dioxan gelöst und mit 1,6 g Raney-Nickel mittlerer Aktivität versetzt. Anschließend wird Wasserstoff bis zu einem Druck von 10 MPa aufgepreßt und die p-Nitrophenylacetat enthaltende Lösung unter Rühren 4 Stunden bei Raumtemperatur umgesetzt. Der H₂-Druck wird durch Zugabe von H₂ konstant gehalten. Anschließend entfernt man den Hydrierkatalysator mittels Filtration und engt die angefallene organische Lösung im Vakuum bei Raumtemperatur ein. Man erhält 6,9 g eines 95 %igen p-Aminophenylacetats (4-Aminophenylacetat) in Form sandfarbener Kristalle (entsprechend 98 % Ausbeute). p-Nitrophenylacetat hat sich vollständig umgesetzt. Nach Umkristallisieren in Toluol/Petrolether fallen 6,07 g p-Aminophenylacetat in einer Reinheit > 99 % (entsprechend 91 % Ausbeute) an.
Umsatz (p-Nitrophenylacetat): 100 %
Selektivität zu p-Aminophenylacetat: 98 %
Ausbeute nach Umkristallisieren: 91 %

### Vergleichsbeispiel 1

Man arbeitet wie in Beispiel 1 angegeben, führt die Reaktion bei 1 MPa H₂-Druck durch.
Reaktionszeit: 24 Stunden
Umsatz (p-Nitrophenylacetat): 58 %
Selektivität zu p-Aminophenylacetat: 84 %

### Vergleichsbeispiel 1a

Man arbeitet wie in Beispiel 1 angegeben, verwendet jedoch (anstelle von Dioxan und Isopropanol) 90 ml Ethanol als Lösungsmittel.
Umsatz (p-Nitrophenylacetat): 100 %
Selektivität zu p-Aminophenylacetat: 68 % (25 % p-Acetamidophenol)

### Beispiel 2

Man arbeitet wie in Beispiel 1 angegeben, verwendet jedoch (anstelle von Dioxan und Isopropanol) 90 ml Isopropanol als Lösungsmittel.
Umsatz (p-Nitrophenylacetat): 100 %
Selektivität zu p-Aminophenylacetat: 85 % (13 % p-Acetamidophenol)

### Vergleichsbeispiel 2

Man arbeitet wie in Beispiel 2 angegeben, verwendet jedoch (anstelle von Dioxan und Isopropanol) 90 ml Isopropanol als Lösungsmittel und führt die Reaktion bei 1 MPa H₂-Druck durch.
Umsatz (p-Nitrophenylacetat). 45 %
Selektivität zu p-Aminophenylacetat: 67 % (4 Nebenprodukte)

### Beispiel 3

Man arbeitet wie in Beispiel 1 angegeben, verwendet jedoch (anstelle von Raney-Nickel) 1,91 g eines Palladium-Träger-Katalysators (5 % Pd auf Aktivkohle).
Umsatz (p-Nitrophenylacetat): 100 %
Selektivität zu p-Aminophenylacetat: 96 %

### Vergleichsbeispiel 3

Man arbeitet wie in Beispiel 3 angegeben, verwendet jedoch (anstelle von Dioxan und Isopropanol) 100 ml Toluol als Lösungsmittel und führt die Reaktion bei 1 MPa H₂-Druck durch.
Reaktionszeit: 1 Stunde
Umsatz (p-Nitrophenylacetat): 0 %

### Beispiel 4

Man arbeitet wie in Beispiel 1 angegeben, verwendet jedoch (anstelle von Dioxan und Isopropanol) 90 ml Toluol als Lösungsmittel.
Umsatz (p-Nitrophenylacetat): 95 %
Selektivität zu p-Aminophenylacetat: 84 % (3 Nebenprodukte)

### Beispiel 5

Man arbeitet wie in Beispiel 1 angegeben, verwendet jedoch (anstelle von Dioxan und Isopropanol) 60 ml Methanol und 30 ml Tetrahydrofuran als Lösungsmittel.
Umsatz (p-Nitrophenylacetat): 100 %
Selektivität zu p-Aminophenylacetat: 96 % (4 % p-Acetamidophenol)

### Beispiel 6

Man arbeitet wie in Beispiel 1 angegeben, verwendet jedoch (anstelle von 30 ml Dioxan und 60 ml Isopropanol) 50 ml Dioxan und 40 ml Isopropanol.
Umsatz (p-Nitrophenylacetat): 100 %
Selektivität zu p-Aminophenylacetat: 96 % (4 % unbekanntes Nebenprodukt)

### Beispiel 7

Man arbeitet wie in Beispiel 1 angegeben, verwendet jedoch (anstelle von Raney-Nickel) 1,0 g eines Nickelträger-Katalysators, der 52 bis 55 Gew.-% Ni und 25 bis 30 Gew.-% Kieselgur als Träger enthält (Handelsprodukt der Hoechst AG; Bezeichnung Ni 55/5 TS) Wasserstoffaufnahme: 1 Stunde; Reaktionszeit insgesamt 4 Stunden
Umsatz (p-Nitrophenylacetat): 100 %
Selektivität zu p-Aminophenylacetat: 86 %

### Beispiel 8

Man arbeitet wie in Beispiel 1 angegeben, verwendet jedoch (anstelle von p-Nitrophenylacetat) 2-Methyl-4-nitrophenylacetat als Einsatzstoff.
Umsatz (2-Methyl-4-nitrophenylacetat): 100 %
Selektivität zu 2-Methyl-4-aminophenylacetat: 97 %

## Patentansprüche

1. Verfahren zur Herstellung eines Aminophenylacetats oder Aminophenyldiacetats, dadurch gekennzeichnet, daß man ein Nitrophenylacetat oder ein Nitrophenyldiacetat in Gegenwart eines Hydrierkatalysators mit Wasserstoff bei 0 bis 60 °C und wenigstens 4 MPa H₂-Druck in Anwesenheit eines Lösungsmittels oder Lösungsmittelgemisches umsetzt.

2. Verfahren nach Anspruch 1, dadurch gekennzeichnet, daß man als Nitrophenylacetat ein gegebenenfalls am aromatischen Kern einfach oder mehrfach durch eine Alkylgruppe mit 1 bis 12, insbesondere 1 bis 4 Kohlenstoffatomen, ein Halogenatom, eine Hydroxygruppe, eine Alkylethergruppe mit 1 bis 4 Kohlenstoffatomen im Alkylrest, eine Arylethergruppe, eine Nitrilgruppe, eine RS-, R₃Si- oder R-CO-Gruppe, worin R für einen Alkylrest mit 1 bis 4 Kohlenstoffatomen oder eine Arylethergruppe steht, durch eine Sulfonsäuregruppe, eine Phosphonsäuregruppe, eine Sulfoxidgruppe, eine Carbonsäuregruppe und/oder Carbonsäuresalzgruppe substituiertes Nitrophenylacetat einsetzt.

3. Verfahren nach Anspruch 1 oder 2, dadurch gekennzeichnet, daß man als Nitrophenylacetat ein gegebenenfalls am aromatischen Kern einfach oder mehrfach durch eine Alkylgruppe mit 1 bis 12, insbesondere 1 bis 4 Kohlenstoffatomen, ein Halogenatom, eine Hydroxygruppe, eine Alkylethergruppe mit 1 bis 4 Kohlenstoffatomen im Alkylrest, eine Arylethergruppe, eine Nitrilgruppe, eine R-CO-Gruppe, worin R für einen Alkylrest mit 1 bis 4 Kohlenstoffatomen steht, oder eine Carbonsäuregruppe, insbesondere durch eine Alkylgruppe mit 1 bis 12, bevorzugt 1 bis 4 Kohlenstoffatomen, ein Halogenatom, eine Alkylethergruppe mit 1 bis 4 Kohlenstoffatomen im Alkylrest, eine Nitrilgruppe oder eine R-CO-Gruppe, worin R für einen Alkylrest mit 1 bis 4 Kohlenstoffatomen steht, bevorzugt durch eine Alkylgruppe mit 1 bis 12, insbesondere 1 bis 4 Kohlenstoffatomen oder eine Alkylethergruppe mit 1 bis 4 Kohlenstoffatomen im Alkylrest substituiertes Nitrophenylacetat einsetzt.

4. Verfahren nach einem oder mehreren der Ansprüche 1 bis 3, dadurch gekennzeichnet, daß man ein gegebenenfalls am aromatischen Kern einfach oder mehrfach substituiertes o-Nitrophenylacetat, m-Nitrophenylacetat und/ oder p-Nitrophenylacetat, insbesondere o-Nitrophenylacetat und/ oder p-Nitrophenylacetat, bevorzugt p-Nitrophenylacetat einsetzt.

5. Verfahren nach einem oder mehreren der Ansprüche 1 bis 4, dadurch gekennzeichnet, daß man als Nitrophenylacetat, o-Nitrophenylacetat, m-Nitrophenylacetat, p-Nitrophenylacetat und/oder 2-Methyl-4-nitrophenylacetat, insbesondere p-Nitrophenylacetat und/oder 2-Methyl-4-nitrophenylacetat einsetzt.

6. Verfahren nach einem oder mehreren der Ansprüche 1 bis 5, dadurch gekennzeichnet, daß der Hydrierkatalysator Kupfer, Mangan, Cobalt, Nickel, Rhodium, Platin und/oder Palladium, insbesondere Cobalt, Nickel, Platin und/oder Palladium, bevorzugt Nickel und/oder Palladium und gegebenenfalls geeignete Aktivatoren und Promotoren enthält.

7. Verfahren nach einem oder mehreren der Ansprüche 1 bis 6, dadurch gekennzeichnet, daß man als Hydrierkatalysator einen trägerfreien Katalysator oder einen Trägerkatalysator einsetzt.

8. Verfahren nach einem oder mehreren der Ansprüche 1 bis 7, dadurch gekennzeichnet, daß man als trägerfreien Katalysator Raney-Cobalt oder Raney-Nickel, insbesondere Raney-Nickel einsetzt.

9. Verfahren nach einem oder mehreren der Ansprüche 1 bis 7, dadurch gekennzeichnet, daß der Trägerkatalysator Al₂O₃, Tonerde, Bimsstein, SiO₂, Silicagel, Kieselsäure, Kieselgur und/oder Aktivkohle, insbesondere Al₂O₃, Tonerde, Kieselgur und/oder Aktivkohle, bevorzugt Al₂O₃, Kieselgur und/oder Aktivkohle enthält.

10. Verfahren nach einem oder mehreren der Ansprüche 1 bis 9, dadurch gekennzeichnet, daß man die Umsetzung bei 10 bis 50, insbesondere 15 bis 45, bevorzugt 20 bis 40 °C durchführt.

11. Verfahren nach einem oder mehreren der Ansprüche 1 bis 10, dadurch gekennzeichnet, daß man die Umsetzung bei 4 bis 100, insbesondere 5 bis 50, bevorzugt 5 bis 30 MPa H₂- Druck durchführt.

12. Verfahren nach einem oder mehreren der Ansprüche 1 bis 11, dadurch gekennzeichnet, daß man als Lösungsmittel einen geradkettigen und/oder verzweigten, einwertigen und/oder mehrwertigen aliphatischen Alkohol, insbesondere einen verzweigten einwertigen, aliphatischen Alkohol, ein aliphatisches Keton, einen nichtcyclischen und/oder cyclischen aliphatischen Ether, einen Alkylester aliphatischer Carbonsäuren, ein Alkylbenzol und/oder ein Dialkylamid einer aliphatischen Carbonsäure einsetzt.

13. Verfahren nach einem oder mehreren der Ansprüche 1 bis 12, dadurch gekennzeichnet, daß man als Lösungsmittel einen aliphatischen Alkohol mit 3 bis 8 Kohlenstoffatomen, insbesondere Isopropanol und/oder sek.-Butanol und/ oder tert. Butanol, ein aliphatisches Keton mit 3 bis 8 Kohlenstoffatomen, insbesondere Aceton und/oder Methylethylketon, einen nichtcyclischen aliphatischen Ether mit 2 bis 4 Kohlenstoffatomen je Alkylgruppe, insbesondere Diethylether und/oder Di-n-butylether, einen cyclischen aliphatischen Ether mit 4 bis 5 Kohlenstoffatomen im Ring, insbesondere Tetrahydrofuran und/oder Dioxan, einen Alkylester einer aliphatischen Carbonsäure mit 1 bis 4 Kohlenstoffatomen, insbesondere einen Methylester oder Ethylester der Ameisensäure und/oder Essigsäure, ein Alkylbenzol wie Toluol, Xylol und/oder Isopropylbenzol, insbesondere Toluol und/oder ein Dialkylamid einer aliphatischen Carbonsäure mit 1 bis 4 Kohlenstoffatomen, insbesondere Dimethylformamid, Dimethylacetamid und/oder N-Methylpyrrolidin einsetzt.

14. Verfahren nach einem oder mehreren der Ansprüche 1 bis 13, dadurch gekennzeichnet, daß man als Lösungsmittelgemisch ein protisch polares Lösungsmittel, insbesondere einen geradkettigen oder verzweigten aliphatischen Alkohol mit 1 bis 4 Kohlenstoffatomen und ein aprotisch polares Lösungsmittel, insbesondere Tetrahydrofuran und/oder Dioxan einsetzt.

15. Verfahren nach einem oder mehreren der Ansprüche 1 bis 14, dadurch gekennzeichnet, daß man als Lösungsmittelgemisch 5 bis 95, insbesondere 10 bis 90, bevorzugt 20 bis 80 Gewichtsteile protisch polares Lösungsmittel und 95 bis 5, insbesondere 90 bis 10, bevorzugt 80 bis 20 Gewichtsteile aprotisch polares Lösungsmittel einsetzt.
